# EUROPÄISCHE PATENTSCHRIFT

(11) **EP 1 984 722 B1**
(45) Veröffentlichungstag und Bekanntmachung des Hinweises auf die Patenterteilung: **17.03.2010**
(21) Anmeldenummer: 07712083.0
(22) Anmeldetag: 23.01.2007
(51) Int. Cl.: G01N 3/40, G01N 33/34

(54) **VERFAHREN UND VORRICHTUNG ZUR BESTIMMUNG DER WEICHHEIT VON HYGIENEPAPIEREN UND TEXTILIEN**
METHOD AND APPARATUS FOR DETERMINING THE SOFTNESS OF HYGIENIC PAPERS AND TEXTILES
PROCEDE ET DISPOSITIF DE DETERMINATION DE LA DOUCEUR DE PAPIERS HYGIENIQUES ET DE TEXTILES

(30) Priorität: 15.02.2006 DE 102006007678; 22.08.2006 US 823099 P
(43) Veröffentlichungstag der Anmeldung: 29.10.2008
(73) Patentinhaber: Grüner, Alexander, 04328 Leipzig (DE)
(72) Erfinder: GRÜNER, Giselher, 04129 Leipzig (DE)
(74) Vertreter: Schneider, Henry
(86) Internationale Anmeldenummer: PCT/EP2007/050628
(87) Internationale Veröffentlichungsnummer: WO 2007/093484

(56) Entgegenhaltungen:
- DE-A1- 19 521 427
- DE-A1- 19 543 674
- US-A- 3 060 719
- US-A- 3 683 681
- HOLLMARK HOLGER ET AL: "Measurement of tissue paper softness: A literature review" NORD PULP PAP RES J; NORDIC PULP AND PAPER RESEARCH JOURNAL 2004, Bd. 19, Nr. 3, 2004, Seiten 345-353, XP008079233

## Beschreibung

Die Erfindung betrifft ein Verfahren und eine Vorrichtung zur Bestimmung der Weichheit von Hygienepapieren und Textilien.

Der Begriff Hygienepapier umfasst Zollstoffwatte, Tissue and Kreppapier und ist zum größten Teil aus Zellstoff und zum geringen Teil aus Altpapierstoff oder mit Holzstoffbeimischungen hergestellt. Die hohe Bedeutung, die Tissue inzwischen erreicht hat, hat dazu geführt, dass sich diese Bezeichnung im internationalen Sprachgebrauch als. Sammelbezeichnung für Hygienepapiere eingebürgert hat. Sie werden zur Herstellung von Toilettenpapier und zahlreicher anderer Hygieneprodukte, wie Taschentücher, Küchentücher, Handtücher und Kosmetiktücher, verwendet.

Hygienepapiere stellen nur etwa 6 % der insgesamt in Deutschland produzierten Papiermengen, sie haben aber eine große Bedeutung für die Lebensqualität der Bevölkerung und entsprechend groß ist die Nachfrage nach Produkten aus diesen Papieren. Im Vordergrund stehen dabei die so genannten Tissue-Papiere, deren Merkmal eine sehr lockere und voluminöse Blattstruktur ist. Zur Erhöhung des Volumens werden sie gekreppt und -um bestimmte technische Eigenschaften zu optimieren - geprägt. Tissue-Papiere werden ein- oder mehrlagig zu Taschentüchern, Toilettenpapieren oder Küchenrollen konfektioniert. Die entsprechenden Produkte müssen eine Reihe von spezifischen Anforderungen erfüllen. Sie müssen beispielsweise hinreichend nassfest sein, ein Mindestmaß an mechanischer Festigkeit besitzen, physiologisch unbedenklich und eben weich sein.

Die Weichheit eines Tissue-Papiers ist eine subjektiv wahrgenommene Eigenschaft, die auch in der industriellen Praxis nach bestimmten, nicht einheitlich festgelegten Methoden sensorisch bestimmt und bewertet wird.

Weichheit ist eine ausschließlich subjektiv wahrnehmbare Eigenschaft, die nicht einmal in eindeutigen naturwissenschaftlichen Begrifflichkeiten zu beschreiben ist. Der Grund dafür ist, dass das subjektive Weichheitsempfinden aus dem Zusammenspiel vieler Produkt-merkmale entsteht. Dieses komplexe Zusammenwirken soll im Folgenden näher erläutert werden.

Weichheit ist in erster Linie eine taktile, also vom Tastsinn wahrgenommene Eigenschaft. Dabei sind folgende Fälle zu unterscheiden:
- Wahrnehmung der Weichheit durch Abtasten der Oberfläche
   Bei dieser Verfahrensweise gleiten die tastenden Fingerkuppen über das plan ausliegende Produkt. Beeinflussend für die Wahrnehmung ist die Oberflächenstruktur des Produktes und die Beschaffenheit des von der Fingerkuppe erzeugten Stauberges. Letzteres wird sicher erheblich von dem Porenvolumen und der Viskoelastizität des Produktes bestimmt. Auch freie, aus der Oberfläche herausragende Faserenden werden die Oberflächenweichheit mit beeinflussen.
- Wahrnehmung der Weichheit durch Falten und/oder Knüllen
   Im Gebrauch werden Tissue-Produkte gefaltet und/oder geknüllt, wobei das Weichheitsempfinden hier sicher wesentlich weniger von den Oberflächeneigenschaften als vielmehr von den Volumeneigenschaften dominiert wird. Das Porenvolumen des Blattes wird ebenso wie die Viskoelastizität Einflussgröße sein.

Die Weichheit des Tissue-Produktes wird nicht nur vom Tastsinn beurteilt. Auch die anderen Sinnesorgane sind von Bedeutung. Werden bei der tastenden Weichheitsbewertung von Tissue beispielsweise knisternde Geräusche erzeugt, wird dieses sicher unbewusst als weichheitsmindernd registriert werden. In gleicher Weise werden gegebenenfalls auch insbesondere unangenehme oder artfremde Gerüche Einfluss auf das Beurteilungsergebnis nehmen.

Die zur Zeit bekannten Mess- und Bewertungsverfahren zur Weichheit werden im Nachfolgenden kurz beschrieben.

Gegenwärdger Stand der Technik ist die Bewertung der Weichheit von Tissue und Tissue-Produkten sensorisch mit Hilfe eines "Panels" - Gruppe mit einer vorgebbaren Anzahl von Personen - gut ausgebildeter Prüfpersonen, mittels eines so genannten "Panel-Tests". Die sensorische Prüfung wird durchgängig von allen Herstellern und Verarbeitern angewendet. Bestandteil der Prüfungen sind aber stets die bereits oben erwähnte tastende Bewertung der Oberflächenbeschaffenheit und die Bewertung des Falt- bzw. Knüllverhaltens. Vorgehensweise und Bewertungskriterien sind unterschiedlich. Weichheitsbewertungen sind deshalb nicht von Hersteller zu Hersteller übertragbar und sehr zeitaufwendig und wegen des Personalaufwandes auch konstenintensiv.

Ferner ist ein so genanntes "Kawabata"-Bewertungssystem [C.M.Carr und J.C.Roberts, "Technology transfer - a quality control toll from textile industry", Paper technology, Nr 11, 1993, S. 27-28] ist für textile Materialien entwickelt worden. Das System besteht aus der Messung einer Vielzahl von Parametern, aus denen auf komplexe und hier nicht näher erläuterte Weise die Bewertung abgeleitet wird. Gemessen werden Zugfestigkeit, Scherfestigkeit, Biegesteifigkeit, Kompressibilität, Dicke und flächenbezogene Masse sowie die Oberflächenbeschaffenheit. Dieser Test ist sehr aufwendig und führt nur bedingt zu einer Reproduzierbarkeit der Weichheit.

Ein von H. Hollmark ["Evaluation of tissue paper softness", Tappi Journal, 1983, Nr. 2, S. 97-99] vorgeschlagenes Verfahren mit der Bezeichnung "Surface Softness Analyzer" zur Bewertung der Oberflächenweichheit funktioniert auf der Basis eines Schallplattenspielers. Auf den Plattenteller wird eine Probe des Tissue-Papiers aufgelegt und für die Dauer von etwa 15 Sekunden in Rotation, versetzt. In dieser Zeit werden mittels eines modifizierten "Tonarms" die Amplitude und die Frequenz der Oberflächenrauigkeit gemessen. Aus beiden Messgrößen wird eine Kennzahl für die Oberflächenweichheit abgeleitet.

Ähnliche Ansätze verfolgen die Druckschriften US 3,026,726 und die DE 30 20 348 A1, die ebenfalls zur Messung der Oberflächengüte von Papieren einen Plattenspieler verwenden.

Bekannt ist auch ein System mit der Bezeichnung "Handle-O-Meter". Beim Handle-O-Meter wird eine Tissue-Probe über eine Nut definierter Breite gelegt. Ein Schwert drückt die Probe in die Nut. Die dafür notwendige Kraft wird gemessen. Diesem Grundgedanken folgt auch die Patentschrift US 3,151,483.

Mit einem weiterer Untersuchungsmethode wartet die Literatur unter der Bezeichnung "WABY-Faktor" [R.S.Ampulsiki, W.U.Spendel, A.H.Sawdai und B.Weistein, "methods for measurement of the mechanical properties of tissue paper", Intemation Paper Physics Conference, Tappi Proceedings, 1991, S. 19-22] auf. Darunter versteht man den Quotienten aus der Probenflexibilität und der Bruchkraft. Die Flexibilität ist ihrerseits definiert durch den Quotienten der Zugkraft (20 N/m) und der dadurch hervorgerufenen Dehnung. In der Literatur wird die Flexibilität in einer gewissen Beziehung zur Weichheit gesehen.

Ferner wird in [R.S.Ampulsiki, W.U.Spendel, A.H.Sawdai und B.Weistein, "methods for measurement of the mechanical properties of tissue paper", Internation Paper Physics Conference, Tappi Proceedings, 1991, S.19-22], die so genannte physiologische Oberflächenweichheit, als ein Faktor definiert, der gewonnen wird, indem eine Tissue-Probe in Maschinenrichtung mit einem Profilometer abgetastet wird. Das gewonnene Signal wird fouriertransformiert, so dass eine Amplituden-Frequenz-Darstellung entsteht, die noch geeignet gefiltert werden muss, um an die subjektive taktile Empfindung angepasst zu werden. Schließlich wird die Fläche unter der gefilterten Kurve integriert. Das Integral liefert den so genannten PSS-Faktor, der mit dem Weichheitsempfinden korrelieren soll.

Letztlich ist zur messtechnischen Bewertung der Weichheit eine Untersuchung mit dem "Kreispendel nach Bekk" möglich. Es handelt sich hierbei um ein Kreispendel, welches zum Gebrauch auf die zu untersuchende Probe aufgesetzt und so weit aus der Ruhelage herausgedreht wird, bis der Anschlag die Probenoberfläche berührt. Wird nun das Pendel freigegeben, schwingt es um die Ruhelage mit abnehmender Schwingungsweite. Gemessen wird die Zeit bis zum vollständigen Abklingen der Schwingung.

Ein Verfahren, bei dem zwei aufgespannte Papierproben gegeneinander gerieben werden, wird in US 3060719 beschrieben. Das entstehende Geräusch wird durch ein Mikrofon gemessen.

Im Ergebnis sind die bekannten Methoden sehr aufwendig und kostenintensiv. Der "Panel-Test" führt zwar zu Ergebnissen hinsichtlich der Weichheit, diese sind jedoch nur bedingt objektiv und führen vor allem zu keinem herstellerunabhängigen vergleichbaren. Messergebnis. Alle anderen Verfahren, insbesondere das "Kawabata"-Bewertungssystem und das "Kreispendel nach Bekk" korrelieren nur ungenügend mit dem subjektiven Empfinden der Verbraucher bzw. differieren nicht genügen und konnten sich somit in der Praxis ebenfalls nicht durchsetzen.

Die für die Ermittlung der Weichheitsfaktoren erforderliche sehr hohe Aufwand und/oder der erforderliche Zeitraum bis ein Ergebnis vorliegt, machen ein schnell messendes und mit der subjektiven Weichheit korrelierendes Bewertungsverfahren für den routinemäßigen Einsatz in der Praxis sehr wünschenswert. Insbesondere soll die Weichheit zwischen verschiedenen Herstellern der Hygienepapiere und/oder Textilien möglichst standardisiert miteinander vergleichbar gemacht werden.

Die Aufgabe der Erfindung besteht also darin, eine Vorrichtung und ein zugehöriges Verfahren anzubieten, die/das in der Lage ist, die Weichheit von Hygienepapier und/oder Textilien schnell und reproduzierbar zu messen.

Die Aufgabe wird nach Anspruch 1 und nach Anspruch 2 gelöst.

Die Vorrichtungen beider Ausführungsvarianten ermöglichen die Durchführung eines erfindungsgemäßen Verfahrens nach Anspruch 20.

Die beiden Ausführungsvarianten zeichnen sich in bevorzugter Ausgestaltung der Erfindung dadurch aus, dass der Schwingungssensor am Element oder im Bereich einer Halteeinrichtung der Probe angeordnet ist.

Die Probe ist auf der Halteeinrichtung unter Ausbildung einer im Wesentlichen ebenen Probenebene angeordnet, wobei die Halteeinrichtung als ein Messgehäuse ausgeführt sein kann, auf dem die Probe mittels mindestens eines Halteelementes befestigbar ist.

In bevorzugter Ausgestaltung der Erfindung weist das Messgehäuse mindestens eine Öffnung auf, auf der die Probe befestigt ist, wobei das Messgehäuse im Weiteren beliebig teilweise offen oder geschlossen ausgebildet ist, so dass für ein sich zwischen Element und Probe ausbildendes Schallfeld entweder ein offener Schallbereich oder ein begrenzter Schallraum ausbildbar ist, in dessen Bereich der Schwingungssensor angeordnet ist.

Der Schwingungssensor kann dabei außerhalb oder im Bereich der Halteeinrichtung oder außerhalb oder im Bereich des Messgehäuses angeordnet werden.

Zur Justierung und Messung der Eindringkraft des Elementes ist in weiterer bevorzugter Ausgestaltung der Erfindung eine Kraftmesseinrichtung angeordnet, die in Abhängigkeit der Kraftrichtung der Eindringkraft im Bereich der Halteeinrichtung der Probe angeordnet ist.

Die Kraftmesseinrichtung kann zur Justierung und Messung der Eindringkraft des Elementes in anderer Ausgestaltung der Erfindung auch am Element selbst oder im Bereich des Elementes angeordnet werden.

Die Anordnung der Kraftmesseinrichtung ist so zu gestalten, dass in Abhängigkeit der ersten oder zweiten Ausführungsvariante, bei der einmal das bewegliche Element zur lagefesten Probe und ein anderes mal die bewegliche Probe zum lagefesten Element geführt wird, eine genaue Messung der Eindringkraft in die Probe ermöglicht wird.

Zur Messung des Verstellweges und somit des Eindringeweges in die Probe ist eine Verstelleinrichtung angeordnet.

Die Anordnung der Verstelleinrichtung ist so zu gestalten, dass in Abhängigkeit der ersten oder zweiten Ausführungsvariante, bei der einmal das bewegliche Element zur lagefesten Probe und ein anderes Mal die bewegliche Probe zum lagefesten Element geführt wird, eine genaue Messung des Verstellweges in die Probe ermöglicht wird.

Der Verstellweg ist ein Maß für die Elastizität der Probe und kann je nach Material vorgegeben und ausgewertet werden. Bei gleichbleibendem Verstellweg ist je nach Material eine unterschiedliche Eindringkraft notwendig, um die Probe um ein gewünschtes Maß zu verformen.

Ferner ist zu beachten und für beide Ausführungsvarianten gleichermaßen anwendbar, dass die Eindringkraft des Elementes in jedem Winkel zur Probe, vorzugsweise orthogonal zur im Wesentlichen horizontalen Probenebene wirkt.

Die eigentliche Relativbewegung zwischen Element und Probe zur Erzeugung der Geräusche ist rotatorisch

Der jeweils bewegliche Teil der Vorrichtung, das Element oder die Probe, ist einerseits zur Realisierung der Relativbewegung zwischen Element und Probe und andererseits zur Realisierung der Verstellbewegung und zur Justierung der Eindringkraft mit einem mechanischen oder elektrischen oder pneumatischen oder hydraulischen Antrieb verbunden, der direkt oder indirekt über ein Übertragungsmittel mit dem Element oder der Probe verbunden sein kann.

Ferner ist in bevorzugter Ausgestaltung der Erfindung der Schwingungssensor ein Mikrofon, welches mit einer Auswerte- und Kalibiereinheit verbunden ist, wobei das Mikrofon und/oder die Auswerte- und Kalibiereinheit jeweils im Bereich der Halteeinrichtung bzw. außerhalb oder innerhalb des Messgehäuses angeordnet ist.

Die Vorrichtung zeichnet sich zudem dadurch aus, dass im Messbereich, im Bereich der Halteeinrichtung oder im Messgehäuse oder in einer die Vorrichtung umgebenden Klimakammer, ein Temperatursensor und/oder ein Feuchtesensor zur Bestimmung der Temperatur und/oder der relativen Feuchte angeordnet ist.

Die Formgebung des Elementes, vorzugsweise des Schaberelementes, kann verschiedene Formen aufweisen. Dabei sind erfindungsgemäß ebene oder konkave oder konvexe oder spitze Anlageflächen an der Probe ausbildbar.

Das Element.kann als Bauteilkörper eine ebene Platte sein. Diese Platte kann ferner mit zur Probe gerichteten konturgebenden Elementen ausgebildet sein, so dass gegenüber der Probenebene die verschiedenen Formen eben, konkav, konvex oder spitz mit den daraus entstehenden Anlageflächen ausbildbar sind.

Ferner ist auf der zur Probe gerichteten Fläche des Elementes eine definierte Oberflächenrauhigkeiten vorgebbar.

Diese Oberflächenrauigkeit ist auch dadurch erreichbar, indem auf der zur Probe gerichteten Fläche des Elementes bezüglich des Materials der Probe gleichartige oder abweichende Hygienepapier- oder Textilmuster angeordnet werden, wodurch ebenfalls definierte Oberflächenrauigkeiten vorgebbar sind.

Schließlich besteht das Element, vorzugsweise das Schaberelement, aus verschiedenen Materialien in geeigneter Mindesthärte, wobei vorzugsweise Kunststoffe oder Metalle mit verschiedenen Oberflächenrauigkeiten einsetzbar sind.

Eine weitere Besonderheit beider Ausführungsvarianten der Erfindung besteht darin, dass die Probe auf der Öffnung der Halteeinrichtung bzw. dem Messgehäuse unter Ausbildung der im Wesentlichen ebenen Probenebene derart angeordnet ist, dass die Probe auf einer geschlossen oder teilweise mit Öffnungen versehenen Folie oder Platte mit unterschiedlich ausführbaren Materialstärken und/oder Materialeigenschaften zur Auflage kommt.

Das mit beiden Vorrichtungen ausführbare Verfahren zeichnet sich dadurch aus, dass die Schwingungsanalyse über ein Schallspektrum oder ein Frequenzband unter Auswertung der Schallintensität und/oder des Schallpegels des Schallsdrucks in vorgebbaren Bereichen des Schallspektrums oder des Frequenzbandes vorgenommen wird und daraus eine komplexe Kennzahl berechnet wird, die mit der Weichheit der Probe korreliert.

Vor der Messung zur Bestimmung der Weichheit wird in bevorzugter Ausgestaltung der Erfindung eine Kalibrierung derart vorgenommen, dass in einem vorgebbaren Bereich des Schallspektrums oder des Frequenzbandes einer bestimmten Weichheit ein bestimmtes Referenz-Schallspektrum oder ein bestimmtes Referenz-Frequenzband zugeordnet wird, wobei hierfür in weiterer bevorzugter Ausgestaltung zudem eine komplexe - i.S. von verschiedene Parameter der Weichheit der Probe objektiv zusammenfassende - Kennzahl zugeordnet wird.

In bevorzugter Ausgestaltung der Erfindung erfolgt die Auswertung der empfangenen Schallspektren oder Frequenzbänder mittels Fourier-Analyse durch Zerlegung der einzelnen Schwingungen der erzeugten Geräusche.

Insgesamt wird das Verfahren im Wesentlichen in folgenden Schritten durchgeführt. Die Probe wird in der erfindungsgemäßen Vorrichtung (erste oder zweite Ausführungsvariante) im Wesentlichen eben eingespannt und befestigt, anschließend wird die Einwirkung der Kraft unter gleichzeitiger Messung der Verstellbewegung und die Relativbewegung zwischen Probe und Element ebenfalls unter gleichzeitiger Messung gleichzeitig oder nacheinander vorgenommen, wodurch in dem vorgebbaren Messzeitraum das Geräusch durch einen Schwingungssensor empfangen und registriert wird, und schließlich eine Auswertung des Geräusches mittels des Schallspektrums oder des Frequenzbandes unter Ermittlung der Kennzahl errechnet wird. Die Kennzahl K korreliert erfindungsgemäß mit einer Weichheit.

Um die Vergleichbarkeit der Ergebnisse zu gewährleisten, wird die Bestimmung der Weichheit vorzugsweise unter Normbedingungen von einer Temperatur (T=23°C) und einer relativen Feuchte (ω = 50%) durchzuführen sein.

Die durch Versuche ermittelte vorzugsweise zu verwendende Eindringkraft liegt für Tissue-Papier im Bereich von circa 0,01 bis circa 1,5 N, wobei für einlagige Proben vorzugsweise eine Eindringkraft von 0,1 N verwendet wird.

Für reißfestere Hygienepapiere und Textilien können auch Kräfte oberhalb des angegebenen Bereichs von 1,5 N vorgegeben werden.

Die Vorrichtung der ersten und zweiten Ausführungsvariante und das zugehörige schnell auszuführende Verfahren ermöglichen in vorteilhafter Weise eine herstellerunabhängige objektive Bewertung der Weichheit anhand einer auswertbaren Kennzahl.

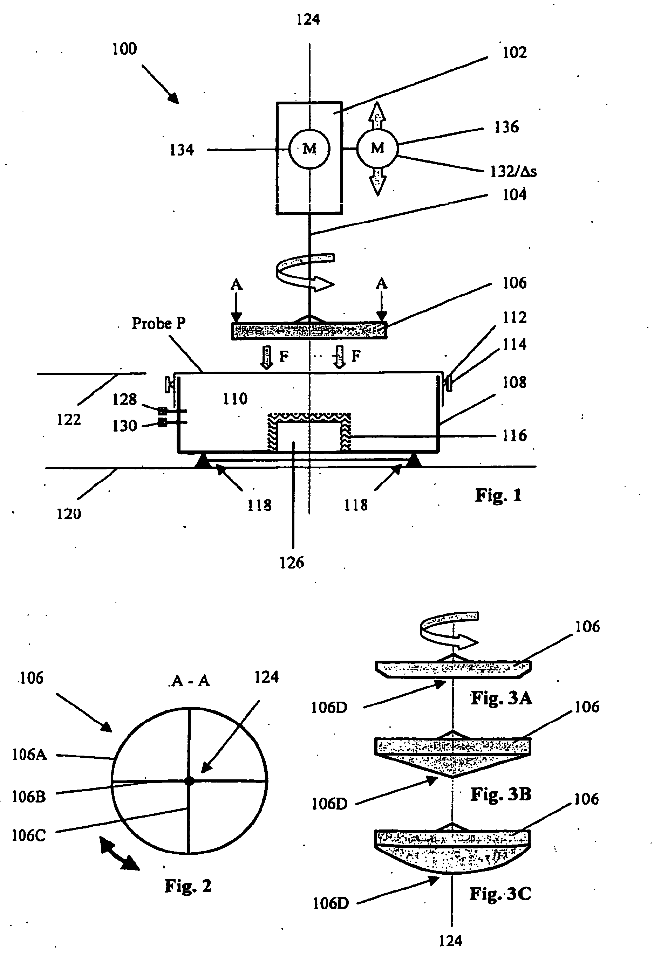

Die Erfindung soll nachfolgend anhand der Figur 1 - erste Ausführungsvariante - in einem Ausführungsbeispiel detailliert beschrieben werden.

Die Figuren 2 und 3A bis 3C ergänzen die erste Ausführungsvariante der Figur 1, können jedoch gleichermaßen für beide Ausführungsvarianten herangezogen werden.

In der Beschreibung der ersten Ausführungsvariante wird teilweise der Unterschied zur zweiten Ausführungsvariante beschrieben, ohne dass die Variante separat dargestellt ist.
Figur 1 zeigt die erfindungsgemäße Vorrichtung 100 zur Bestimmung der Weichheit von Hygienepapieren.
Figur 2 zeigt eine Draufsicht auf ein Element, insbesondere ein Schaberelement 106, welches in beiden Ausführungsvarianten gleichermaßen einsetzbar ist.
Figur 3A bis 3C zeigt verschiedene Formen des Schaberelementes 106, die ebenfalls so in beiden Ausführungsvarianten einsetzbar sind.

Gemäß Figur 1 umfasst die Vorrichtung 100 ein bewegliches Element 106, welches nachfolgend als Schaberelement bezeichnet wird. Dieses Schaberelement 106 ist über ein Übertragungsmittel 104 - im Ausführungsbeispiel eine Antriebswelle - mit einem Antrieb 102 verbunden, der auf einer Achse 124 eine rotatorische Bewegung des Schaberelementes 106 über einen ersten Motor 134, der im Antrieb 102 schematisiert dargestellt ist, bewirkt. Der Antrieb 102 ist mit einem zweiten Motor 136 versehen, der neben dem Antrieb 102 dargestellt ist, so dass das Schaberelement 106 neben der rotatorischen Bewegung um die Achse 124 auch eine entlang der Achse 124 verlaufende Bewegung ausführen kann. Diese Bewegung Δs ist über eine Wegmesseinrichtung 132 vorgebbar und messbar, wobei hieraus ein Parameter ableitbar ist, der einer Elastizität einer Probe P des Hygienepapiers oder Textils entspricht und zur Berechnung der Weichheit benutzt werden kann.

Das Schaberelement 106 ist im vorliegenden Ausführungsbeispiel rotatorisch gegenüber der Probe P und verstellbar -hier höhenverstellbar- gegenüber der Probe P angeordnet Die Verstellung erfolgt also hier vertikal zur horizontal angeordneten Probe P.

Die Eindringkraft F, die auf die Probe P wirkt und ihre beispielhafte Wirkrichtung ist in Figur 1 mittels Pfeilen in Richtung der Probe P dargestellt. Es ist auch eine Wirkrichtung der Eindringkraft F denkbar, die nicht vertikal zur horizontalen Probe P, sondern die in einem vorgebbaren Winkel abweichend von 90° wirkt.

Dem Schaberelement 106 zugeordnet ist im Ausführungsbeispiel ein Messgehäuse 108, welches aus vertikalen Seitenwänden und einer horizontalen Bodenwand aufgebaut ist.

Zur Befestigung der Probe P dienen im Ausführungsbeispiel die Seitenwände des Messgehäuses 108, auf welchem die Probe P des jeweiligen Testmaterials [Hygienepapier oder Textil] beispielsweise durch einfaches Umlegen der Randbereiche der Probe P aufgespannt wird, wobei die Befestigung vorzugsweise über ein Dichtelement 102, insbesondere eine Gummilippe, und mittels einem Halteelement 114 vorzugsweise einer Schelle, einem Gummi oder dergleichen, befestigbar ist. Durch diese Anordnung ergibt sich eine Probenebene 122, die oberhalb eines Messraumes 110 im Messgehäuse 108 angeordnet ist.

Ein solcher Messraum 110 ist für die Erfindung nicht unbedingt notwendig. Die Anordnung der Probenebene 120 könnte auch oberhalb eines offenen Bereiches ohne entsprechende Seitenwände bzw. Bodenwände des Messgehäuses 108 ohne Ausbildung eines abgeschlossenen Messraumes 110 ausgebildet werden.

Vorzugsweise ist jedoch ein solches Messgehäuse 108 unter Ausbildung eines Messraumes 110 vorzusehen, in welchem zur Messung einer Temperatur T bzw. einer relativen Feuchte ω ein Temperatursensor 128 und ein Feuchtesensor 130 angeordnet ist. Die Anordnung bietet sich vorzugsweise in einer der Seitenwände des Messgehäuses 108 an, wobei eine Anordnung selbstverständlich ebenfalls unabhängig vom Messgehäuse 108 ausgeführt werden kann, wenn die entsprechenden Seitenwände nicht ausgebildet sind.

Insgesamt sei erwähnt, dass die Vorrichtung 100 auch in einem Klimaraum oder einer Klimakammer angeordnet werden kann, in der gleichbleibende Temperaturbedingungen bzw. Bedingungen der relativen Feuchte vorherrschen, wobei vorzugsweise zur Standardisierung der Messung die Normalbedingungen, eine Temperatur von T=23 °C und eine relative Feuchte von ω=50 % als Normdaten, die auch allgemein aus dem Stand der Technik bekannt sind, Anwendung finden.

Erfindungsgemäß ist im Inneren des Messgehäuses 108. oder eben im Bereich einer Halteeinrichtung ein Schwingungssensor 116 angeordnet, der mit einer Auswerte- und Kalibriereinheit 126 verbunden ist.

Im Ausführungsbeispiel ist die Auswerte- und Kalibriereinheit 126 im Schwingungssensor 116 angeordnet. Diese Bauteile 116, 120 können jedoch auch separat ausgeführt werden, so dass die dargestellte Anordnung nur beispielhaft ist.

Die Figur 1 zeigt, dass das Messgehäuse 108 auf Kraftmesseinrichtungen 118 angeordnet ist, die gegenüber einem Untergrund 120 fest angeordnet sind und mittels denen eine indirekt auf die Probe P wirkende Eindringkraft F gemessen werden kann.

Der Schwingungssensor 116 ist grundsätzlich jede Art von Messeinrichtung, die in der Lage ist, Schallspektren oder Frequenzbänder zu empfangen und zu registrieren, da innerhalb des erfindungsgemäßen Verfahrens eine Schwingungsanalyse der zwischen dem Schaberelement 106 und der Probe P entstehenden Geräusche durchgeführt wird.

In Figur 2 ist in einer Draufsicht A-A ein möglicher grundsätzlicher Aufbau des Schaberelementes 106 dargestellt, welches aus einem kreisrunden Schabergehäuse 106A mit Schabergehäuseversteifungen 106B und 106C ausgebildet ist. Andere auch nicht kreisrunde Formen sind denkbar.

Die Figuren 3A bis 3B zeigen das Schaberelement 116 in Schnitten durch das Schaberelement 106, wobei deutlich wird, dass verschiedene Schaberformen 106 ausführbar sind. Figur 3A zeigt einen Schaber, der im Wesentlichen wie in Figur 1 ausgeführt ist, jedoch mit reduzierten Eckbereichen eingesetzt wird, wobei das Schaberelement 106 in Figur 3A gegenüber der Probe P eine im Wesentlichen ebene Anlagefläche ausbildet Fehlende Kanten/Ecken ermöglichen die Reduzierung von Zerstörungen der Probe P im Messzeitraum Δt. Die Anlagefläche des Schaberelementes 106, gemäß Figur 3B wird durch ein kegelig spitz zulaufendes Schaberelement 106 bestimmt. Die Schaberform 106D gemäß Figur 3C ist in einer weiteren möglichen Ausführung konvex dargestellt, wodurch sich ebenfalls eine entsprechend charakteristische Anlagefläche auf der Probe P ergibt. Nicht dargestellt, aber ebenfalls ausführbar, ist beispielsweise eine konkave Form des Schaberelementes 106.

Das Schaberelement 116 kann als Bauteilkörper auch nur als ebene Platte ausgebildet werden (nicht dargestellt), so dass auch hier eine ebene Anlagefläche ausgebildet wird.

Vor der Beschreibung des erfindungsgemäßen Verfahrens sei noch einmal darauf hingewiesen, dass die Vorrichtung unter analoger funktioneller Anwendung so ausführbar ist, dass die mindestens einlagige Probe P bewegliche angeordnet werden kann, wobei dann das Schaberelement 106 als lagefestes Teil ausgeführt wird.

Dementsprechend ist dann der Antrieb 102 an der Halteeinrichtung oder an dem ausgebildeten Messgehäuse 108 anzuordnen, so dass sich die Probe P über die Halteeinrichtung, gemäß der vorgesehenen Bewegungsart rotatorische gegenüber dem Schaberelement 106 bewegen kann, wobei zudem sichergestellt werden muss, dass eine Verstellbewegung der Halteeinrichtung bzw. des Messgehäuses 108 der Probe P in Richtung des Schaberelementes 106 zur Vorgabe der Eindringkraft F und/oder des Verstellweges Δs ausgeführt werden muss. Die Kraftmesseinrichtung 118 sitzt dann analog zum ersten Ausführungsbeispiel am lagefesten Schaberelement 116, um die vorgegebene Endringkraft F der beweglichen Probe P einstellen und messen zu können. Die Wegmesseinrichtung 132 ist analog am beweglichen Teil, der Probe P bzw. der Halteeinrichtung der Probe P anzuordnen.

Das erfindungsgemäße Verfahren wird nun anhand der Figur 1 näher erläutert, wobei die Grundprinzipien des Verfahrens auch für die Vorrichtung in der nicht dargestellten zweiten Ausführungsvariante mit beweglicher Probe P und lagefestem Schaberelement 106 gelten.

Das Verfahren zur Bestimmung der Weichheit von Hygienepapieren läuft wie folgt: Aus einer in der Praxis vorhandenen Charge von Hygienepapier oder eines Textils wird eine entsprechende Probe P entnommen und über das Halteelement 114 auf das Messgehäuse 108 gespannt. Das höhenverstellbare Schaberelement 106 wird über den Antrieb 102 zunächst über den zweiten Motor mit einer vorgebbaren Eindringkraft auf die leicht flexible Probe P verbracht. Eine vorzugsweise einstellbare Einwirkkraft F beträgt 0.1 N, wobei zudem ein Verstellweg Δs vorgegebenen und anschließend gemessen und überwacht wird.

Über den Antrieb 102 wird das Schaberelement 106 in Rotation versetzt und erzeugt für einen vorgebbaren Zeitraum Δt ein Geräusch, welches sich wellenartig unter Erzeugung von komplexen Schwingungen im Bereich der Probe P ausbreitet. Eine bevorzugte Rotationsgeschwindigkeit beträgt circa 1 Hz, wobei selbstverständlich auch Geschwindigkeiten darüber und darunter zum Einsatz kommen können, die stets unter der Berücksichtigung, dass die Probe (P) nicht reißt, gewählt werden.

Das Verfahren kann auch so ausgebildet sein, dass die Verstellbewegung Δs und die Rotationsbewegung gleichzeitig beginnen, so dass ein Geräusch hervorgerufen wird, bei dem das Schaberelement 106 während seiner Rotation in die Probe P eindringt.

Diese Schwingungen, die innerhalb bzw. außerhalb des menschlichen Hörbereichs liegen, sind die Grundlage für eine Schwiringungsanalyse und die Ermittlung eines Schallspektrums oder eines Frequenzbandes, wobei im Weiteren die Bezeichnung Schall für Schwingungen verwendet wird, die sowohl innerhalb als auch außerhalb des menschlichen Hörbereichs liegen.

Die Aufnahme der Schwingungen erfolgt durch den Schallsensor 116, der gemäß Figur 1 im Messraum 110 angeordnet ist, in dem ein entsprechendes Schallfeld ausgebildet wird.

Hier wird deutlich, dass selbstverständlich das Geräusch auch außerhalb des Messraumes 110, beispielsweise auch gemäß Figur 1 oberhalb der Probe P aufgenommen werden könnte.

Ein Messraum 110 mit definierten Bedingungen, wie der relativen Feuchte ω=50 % und der Temperatur T=23°C und gleichbleibenden Umgebungsbedingungen (ohne Nebengeräusche etc.) bietet sich jedoch zur Vergleichbarkeit der Messungen an.

Durch die Auswerte- und Kalibriereinheit 126 erfolgt durch Integration eines Computers oder einer Datenverarbeitungseinrichtung oder dergleichen in das erfindungsgemäße System eine Auswertung der empfangenen und registrierten Schwingungen mittels einer Schwingungsanalyse, wobei vorzugsweise eine Schallintensität I und/oder ein Schallpegel Lₚ des Schalldrucks p in vorgebbaren Bereichen des Schallspektrums und/oder Frequenzen des Frequenzbandes vorgenommen wird. Aus diesen Werten lässt sich letztlich vorzugsweise durch eine Fourier-Analyse eine Kennzahl K ermitteln, die mit der Weichheit der Probe P korreliert.

Um letztlich eine Aussage über die Weichheit treffen zu können, wird zuvor einem Referenzschallspektrum oder einem Referenzfrequenzband eine bestimmte Weichheit und eine darauf basierende komplexe Kennzahl K zugeordnet.

Durch diese Referenzschallspektren bzw. Referenzfrequenzbänder können die gemessenen und nachfolgend errechneten Daten einer mit der Weichheit korrelierenden Kennzahl K zugeordnet werden.

Ein zur Auswertung benutzter Computer oder dergleichen kann auch für die entsprechenden Stellbewegungen des Schaberelementes 106 gegenüber der lagefesten Probe P oder umgekehrt (je nach Ausführungsvariante) eingesetzt werden.

Der Computer kann die Rotationsbewegung des Schaberelementes 106 und die Verstellbewegung Δs der Probe P oder umgekehrt und den dafür vorgesehenen Zeitraum Δt steuern und regeln, wobei die vorgebbare Eindringkraft F ebenfalls über den Computer gesteuert oder geregelt werden kann.

Versuche haben gezeigt, dass das subjektive Empfinden von Weichheit, welches durch eine Vielzahl von Parametern, zum Beispiel Glätte, Rauigkeit, Oberflächenweichheit, Kompressibilität und der auftretenden Geräusche beim Knüllen einer Probe P, durch das beschriebene Messverfahren und mittels der dargestellten Vorrichtung 100 messtechnisch objektiv erfasst werden kann, da die Messergebnisse sehr gut mit den subjektiven manuellen Ergebnissen, die hinsichtlich der Weichheit durch Testpersonen durchgeführt werden, korrelieren.

Das Verfahren ist somit herstellerunabhängig zur Bestimmung einer objektiven Weichheit einsetzbar und bietet somit die Möglichkeit einer Standardisierung.

### Bezugszeichenliste

- 100: Vorrichtung
- 102: Antrieb
- 104: Übertragungsmittel [Antriebswelle]
- 106: Element [Schaberelement]
- 106A: Schabergehäuse
- 106B: Schabergehäuseversteifung
- 106C: Schabergehäuseversteifungs
- 106D: Schaberform
- 108: Messgehäuse
- 110: Messraum [Schallfeld]
- 112: Dichtelement [Gummilippe]
- 114: Halteelement
- 116: Schwingungssensor
- 118: Kraftmesseinrichtung
- 120: Untergrund
- 122: Probenebene
- 124: Achse
- 126: Auswerte- und Kalibriereinheit
- 128: Temperatursensor (T) [Temperatur]
- 130: Feuchtesensor (ω) [relative Feuchte]
- 132: Wegmesseinrichtung
- 134: erster Motor
- 136: zweiter Motor
- F: Eindringkraft
- P: Probe
- p: Schalldruck
- f: Frequenz
- 1: Schallintensität
- L: Schalldruckpegel
- K: Kennzahl der Weichheit
- Δt: Messzeitraum
- Δs: Wegdifferenz

## Patentansprüche

1. Vorrichtung (100) zur Bestimmung der Weichheit eines mindestens einlagigen Hygienepapiers oder Textils, wobei
die Vorrichtung ein vertikal auf einer Achse (124) liegendes Schaberelement (106) umfasst, welches relativ zu einer lagefesten, mindestens einlagigen, horizontal liegenden Probe (P) des Hygienepapiers oder Textils drehbeweglich angeordnet ist und das gegenüber der auf einem Messgehäuse (108) aufgespannten Probe (P) unter Vorgabe einer auf die Probe (P) orthogonal wirkenden Eindringkraft (F) entlang der Achse (124) verstellban so ausgeführt ist, dass es unter Verformung der Probe (P) in die Probe (P) eindringt, wobei im Bereich der zwischen Probe (P) und dem Schaberelement (106) entstehenden Schwingungen ein Schwingungssensor (116) angeordnet ist, der die während der um die Achse (124) bewirkten Rotation, des auf die Probe (P) einwirkenden Elementes (106) erzeugten Geräusche registriert.

2. Vorrichtung (100) zur Bestimmung der Weichheit eines mindestens einlagigen Hygienepapiers oder Textils, wobei
die Vorrichtung ein vertikal auf einer Achse (124) liegendes Schaberelement (106) umfasst, welches relativ zu einer drehbeweglichen, mindestens einlagigen, horizontal liegenden Probe (P) des Hygienepapiers oder Textils, lagefest angeordnet ist und wobei die auf einem Messgehäuse (108) aufgespannte Probe (P) unter Vorgabe einer auf die Probe (P) orthogonal wirkenden Eindringkraft (F) des Schaberelementes (106) entlang der Achse (124) verstellbar so ausgeführt ist, dass das Schaberelement (106) unter Verformung der Probe (P) in die Probe (P) eindringt, wobei im Bereich der zwischen Probe (P) und dem Element (106) entstehenden Schwingungen ein Schwingungssensor (116) angeordnet ist, der die während der um die Achse (124) bewirkten Rotation, der auf das Schaberelement (106) einwirkenden Probe (P) erzeugten Geräusche registriert.

3. Vorrichtung nach Anspruch 1 oder 2, wobei
der Schwingungssensor (116) im Schallfeld des Geräusches unabhängig oder an der Vorrichtung (100), vorzugsweise am Schaberelement (106) selbst oder im Bereich einer Halteeinrichtung der Probe (P) angeordnet ist.

4. Vorrichtung nach Anspruch 3, wobei
die Probe (P) auf der Halteeinrichtung unter Ausbildung einer im Wesentlichen ebenen Probenebene (122) angeordnet ist, wobei die Halteeinrichtung im Wesentlichen ein Messgehäuse (108) umfasst, auf dem die Probe (P) mittels mindestens eines Halteelementes (114) befestigbar ist.

5. Vorrichtung nach Anspruch 2 bis 4, wobei
das Messgehäuse (108) mindestens eine Öffnung aufweist, auf der die Probe (P) befestigt ist, wobei das Messgehäuse (108) im Weiteren beliebig teilweise offen oder geschlossen ausgebildet ist, so dass für ein sich zwischen Schaberelement (106) und Probe (P) ausbildendes Schallfeld entweder ein offener Schallbereich oder ein begrenzter Schallraum (110) ausbildbar ist, in dessen Bereich der Schwingungssensor (116) angeordnet ist.

6. Vorrichtung nach Anspruch 3 bis 5, wobei
der Schwingungssensor (116) außerhalb oder im Bereich der Halteeinrichtung oder außerhalb oder im Bereich des Messgehäuses (108) angeordnet ist.

7. Vorrichtung nach Anspruch 1 oder 2, wobei
zur Justierung und Messung der Eindringkraft (F) des Schaberelementes (106) eine Kraftmesseinrichtung (118) angeordnet ist, die in Abhängigkeit der Kraftrichtung der Eindringkraft (F) im Bereich der Halteeinrichtung der Probe (P) angeordnet ist.

8. Vorrichtung nach Anspruch 1 oder 2, wobei
zur Justierung und Messung der Eindringkraft (F) des Schaberelementes (106) eine Kraftmesseinrichtung (118) angeordnet ist, die in Abhängigkeit der Kraftrichtung der Eindringkraft (F) am Schaberelement (106) selbst oder im Bereich des Schaberelementes (106) angeordnet ist.

9. Vorrichtung nach Anspruch 1 oder 2, wobei
zur Messung der Eindringtiefe des Schaberelementes (106) eine Wegmesseinrichtung (132) angeordnet ist, durch die ein Verstellweg (Δs) des Schaberelementes (106) oder der Probe (P) vorgebbar und/oder in Abhängigkeit des Materials der Probe (P) und der Eindringkraft (F) als Parameter für die Elastizität der Probe messbar ist.

10. Vorrichtung nach Anspruch 1 oder 2 oder 7 oder 8, wobei
die Eindringkraft (F) des Schaberelementes (106) in jedem Winkel zur Probe (P), vorzugsweise orthogonal zu einer im Wesentlichen horizontalen Probenebene (122) wirkt.

11. Vorrichtung nach Anspruch 1 oder 2, wobei
das jeweilige bewegliche Teil, das Schaberelement (106) oder die Probe (P) rotatorisch, translatorisch oder pendelartig unter Erzeugung des Geräusches bewegbar angeordnet ist.

12. Vorrichtung nach Anspruch 1 oder 2, wobei
die Relativbewegung des Schaberelementes (106) oder der Probe (P) einerseits und die Verstellbewegung des Schaberelementes (106) oder der Probe (P) zur Justierung der Eindringkraft (F) andererseits durch einen mechanischen oder elektrischen oder pneumatischen oder hydraulischen Antrieb oder dergleichen erfolgt, der direkt oder indirekt über ein Übertragungsmittel (104) mit dem Element (106) oder der Probe (P) verbunden ist.

13. Vorrichtung nach Anspruch 1 oder 2, wobei
der Schwingungssensor (116) ein Mikrofon ist, welches mit einer Auswerte- und Kalibriereinheit (126) verbunden ist, wobei das Mikrofon und/oder die Auswerte- und Kalibriereinheit (126) im Bereich der Halteeinrichtung bzw. innerhalb oder außerhalb des Messgehäuses (108) angeordnet ist.

14. Vorrichtung nach Anspruch 1 bis 4, wobei
im Messbereich, im Bereich der Halteeinrichtung oder im Messgehäuse (108) oder in einer die Vorrichtung (100) umgebenden Klimakammer ein Temperatursensor (128) und/oder ein Feuchtesensor (130) zur Bestimmung der Temperatur (T) und/oder der relativen Feuchte (ω) angeordnet ist.

15. Vorrichtung nach Anspruch 1 oder 2, wobei
das Schaberelement (106) als Bauteilkörper eine ebene Platte aufweist oder mit den zur Probe (P) gerichteten konturgebenden Elementen einstückig oder mehrstückig ausgebildet ist, so dass gegenüber der Probenebene (122) verschiedene Formen (106D) ausbildbar sind, die ebene oder konkave oder konvexe oder spitze Anlageflächen an der Probe (P) ausbilden.

16. Vorrichtung nach Anspruch 15, wobei
auf der zur Probe (P) gerichteten Fläche des Elementes (106) (Anlagefläche) eine definierte Oberflächenrauhigkeiten vorgebbar ist.

17. Vorrichtung nach Anspruch 1, wobei
auf der zur Probe (P) gerichteten Fläche des Schaberelementes (106) (Anlagefläche) bezüglich der Probe (P) gleichartige oder abweichende Hygienepapier- oder Textilmuster angeordnet werden, wodurch ebenfalls eine definierte Oberflächenrauhigkeit vorgebbar ist.

18. Vorrichtung nach Anspruch 1 oder 2, wobei
das Schaberelement (106) aus verschiedenen Materialien geeigneter Mindesthärte besteht, wobei vorzugsweise Kunststoffe oder Metalle mit vorgebbaren Oberflächenrauhigkeiten einsetzbar sind.

19. Vorrichtung nach Anspruch 4, wobei
die Probe (P) auf der Halteeinrichtung unter Ausbildung der im Wesentlichen ebenen Probenebene (122) angeordnet ist, wobei die Probe (P) auf einer geschlossenen oder teilweise mit Öffnungen versehenen Folie oder Platte mit unterschiedlich ausführbaren Materialstärken und/oder Materialeigenschaften zur Auflage kommt.

20. Verfahren zur Bestimmung der Weichheit eines mindestens einlagigen Hygienepapiers oder Textils, wobei
- durch ein, relativ zu einer lagefesten, mindestens einlagigen horizontal, liegenden Probe (P) des Hygienepapiers oder Textils, drehbewegliches, vertikal auf einer Achse (124) liegendes Schaberelement (106), welches mit einer vorgebbaren Eindringkraft (F) orthogonal auf die, auf einem Messgehäuse (108) aufgespannte Probe (P) wirkt und die Probe (P) verformt und in die Probe (P) eindringt,
oder
- durch eine, relativ zu einem vertikal auf einer Achse (124) liegenden, lagefesten Schaberelement (106) drehbewegliche mindestens einlagige horizontal liegende Probe (P) des Hygienepapiers oder Textils, die mit einer vorgebbaren Eindringkraft (F) orthogonal auf das lagefeste Schaberelement (106) wirkt, wodurch das Schaberelement (106) in die auf einem Messgehäuse (108) aufgespannte Probe (P) eindringt und die Probe (P) verformt,
in einem vorgebbaren Messzeitraum (Δt) ein Geräusch hervorgerufen, empfangen und registriert wird, wobei die erzeugten Schwingungen mittels einer Schwingungsanalyse unter Ermittlung eines Schallspektrums oder eines Frequenzbandes ausgewertet werden und jedem ermittelten Schallspektrum oder Frequenzband eine bestimmte Weichheit der Probe (P) zugeordnet wird.

21. Verfahren nach Anspruch 20, wobei
die Schwingungsanalyse über das Schallspektrum oder das Frequenzband unter Auswertung der Schallintensität (I) und/oder des Schallpegels (Lₚ) und/oder der Frequenzen (f) des Schalldrucks (p) in vorgebbaren Bereichen des Schallspektrums und/oder des Frequenzbandes vorgenommen wird und daraus eine komplexe Kennzahl (K) berechnet wird, die mit der Weichheit der Probe (P) korreliert.

22. Verfahren nach Anspruch 20 und/oder 21, wobei
vor der Messung zur Bestimmung der Weichheit eine Kalibrierung derart vorgenommen wird, dass in einem vorgebbaren Bereich des Schallspektrums oder des Frequenzbandes jeweils eine bestimmten Weichheit mindestens einem Referenz-Schallspektrum oder mindestens einem Referenz-Frequenzband und einer daraus errechenbaren komplexen Kennzahl (K) zugeordnet wird.

23. Verfahren nach Anspruch 20 bis 22, wobei
die Auswertung der empfangenen Schallspektren oder Frequenzbänder mittels Fourier-Analyse durch Zerlegung der einzelnen Schwingungen der erzeugten Geräusche durchgeführt wird.

24. Verfahren nach Anspruch 20 bis 22, wobei
- die Einwirkung der Kraft (F) und die Relativbewegung (Δs) zwischen Probe (P) und Schaberelement (106) gleichzeitig oder nacheinander vorgenommen und gemessen wird,
- in dem vorgebbaren Messzeitraum (Δt) das Geräusch empfangen und registriert wird, und
- schließlich eine Auswertung des Geräusches mittels des Schallspektrums oder des Frequenzbandes unter Ermittlung der Kennzahl (K) errechnet wird.

25. Verfahren nach Anspruch 20 bis 24, wobei
eine Bestimmung der Weichheit zur Vergleichbarkeit der Ergebnisse vorzugsweise unter Normbedingungen von einer Temperatur (T=23°C) einer relativen Feuchte (ω = 50 %) durchgeführt wird.

26. Verfahren nach Anspruch 20, wobei
die vorgebbare Eindringkraft (F) für Hygienepapiere im Bereich von circa 0,01 N bis circa 150 N und für Textilien in diesem Bereich und darüber liegt.

## Claims

1. A device (100) for determining the degree of softness of a sanitary paper or textile with at least one layer, wherein
the device comprises a scraper element (106) which lies vertically on an axis (124), which is arranged in such a manner that it can be moved relative to a fixed position, horizontal sample (P) of the sanitary paper or textile with at least one layer, and which is designed so that it can be adjusted opposite the sample (P) which is tensioned opposite on a measurement housing (108) under specification of a penetration force (F) which acts orthogonally on the sample (P) along the axis (124), in such a manner that it penetrates into the sample (P) while deforming the sample (P), wherein in the area where vibrations occur between the sample (P) and the scraper element (106), a vibration sensor (116) is arranged which during the rotation which is created around the axis (124) registers the noises which are generated by the element (106) which impacts on the sample (P).

2. A device (100) for determining the softness of a sanitary paper or textile with at least one layer, wherein
the device comprises a scraper element (106) which lies vertically on an axis (124), which is arranged in a fixed position relative to a sample (P) of the sanitary paper or textile with at least one layer, which lies horizontally in such a manner that it can be rotated, and wherein the sample (P) which is tensioned on a measurement housing (108) is designed so that it can be adjusted under specification of a penetration force (F) of the scraper element (106) which acts orthogonally on the sample (P) along the axis (124) in such a manner that the scraper element (106) penetrates into the sample (P) while deforming the sample (P), wherein in the area where vibrations occur between the sample (P) and the element (106), a vibration sensor (116) is arranged which registers the noises which are generated during the rotation created around the axis (124) by the sample (P) acting on the scraper element (106).

3. A device according to claim 1 or 2, wherein
the vibration sensor (116) is arranged independently in the sound field of the noise or on the device (100), preferably on the scraper element (106) itself or in the area of a holding device of the sample (P).

4. A device according to claim 3, wherein
the sample (P) is arranged on the holding device to form an essentially level sample level (122), wherein the holding device essentially comprises a measurement housing (108), on which the sample (P) can be attached by means of at least one holding element (114).

5. A device according to claims 2 to 4, wherein
the measurement housing (108) comprises at least one opening, on which the sample (P) is attached, wherein the measurement housing (108) is furthermore designed partially as required to be either open or closed, so that for a sound field which is formed between the scraper element (106) and the sample (P), either an open sound area or a restricted sound space (110) can be formed, in the area of which the vibration sensor (116) is arranged.

6. A device according to claims 3 to 5, wherein
the vibration sensor (116) is arranged outside or within the area of the holding device or outside or within the area of the measurement housing (108).

7. A device according to claim 1 or 2, wherein
in order to adjust and measure the penetration force (F) of the scraper element (106), a force measurement device (118) is arranged which depending on the direction of force of the penetration force (F) is arranged in the area of the holding device of the sample (P).

8. A device according to claim 1 or 2, wherein
in order to adjust and measure the penetration force (F) of the scraper element (106), a force measuring device (118) is arranged, which depending on the direction of force of the penetration force (F) is arranged on the scraper element (106) itself or in the area of the scraper element (106).

9. A device according to claim 1 or 2, wherein
in order to measure the penetration depth of the scraper element (106), a path measurement device (132) is arranged, via which a displacement path (Δs) of the scraper element (106) or the sample (P) can be specified and/or measured depending on the material of the sample (P) and the penetration force (F) as a parameter for the elasticity of the sample.

10. A device according to claim 1 or 2 or 7 or 8, wherein
the penetration force (F) of the scraper element (106) acts in every angle to the sample (P), preferably orthogonally to a sample level (122) which is essentially horizontal.

11. A device according to claim 1 or 2, wherein
the respective moving part, the scraper element (106) or the sample (P) is arranged in such a manner that it can be moved in a rotatable, translatory or pendulum-type manner while generating the noise.

12. A device according to claim 1 or 2, wherein
the relative movement of the scraper element (106) or the sample (P) on the one hand and the displacement movement of the scraper element (106) or the sample (P) for adjusting the penetration force (F) on the other is achieved by means of a mechanical or electrical or pneumatic or hydraulic drive or similar, which is directly or indirectly connected via a transfer means (104) to the element (106) or the sample (P).

13. A device according to claim 1 or 2, wherein
the vibration sensor (116) is a microphone which is connected to an evaluation and calibration unit (126), wherein the microphone and/or the evaluation and calibration unit (126) is arranged in the area of the holding device or within our outside the measurement housing (108).

14. A device according to claims 1 to 4, wherein
in the measuring area, in the area of the holding device or in the measurement housing (108), or in a climate chamber which surrounds the device (100), a temperature sensor (128) and/or a humidity sensor (130) is arranged to determine the temperature (T) and/or the relative humidity (ω).

15. A device according to claim 1 or 2, wherein
the scraper element (106) as a component body comprises a level plate, or is designed as a single or multiple piece with the contour-giving elements which are aligned to the sample (P), so that opposite the sample level (122), different forms (106D) can be created which form level or concave or convex or tipped attachment surfaces on the sample (P).

16. A device according to claim 15, wherein
on the surface of the element (106) which is aligned to the sample (P) (attachment surface), a defined surface roughness can be specified.

17. A device according to claim 1, wherein
on the surface of the scraper element (106) which is aligned to the sample (P) (attachment surface), sanitary paper or textile patterns are arranged which are the same or different to the sample (P), as a result of which, a defined surface roughness can also be specified.

18. A device according to claim 1 or 2, wherein
the scraper element (106) consists of different materials of a suitable minimum hardness, wherein preferably, synthetic materials or metals with specified surface roughness can be used.

19. A device according to claim 4, wherein
the sample (P) is arranged on the holding device to form the essentially level sample level (122), wherein the sample (P) is attached to a closed film or plate, or one which is partially provided with openings, with material strengths and/or material properties which can be of different design.

20. A method for determining the softness of a sanitary paper or textile with at least one layer, wherein
- due to a scraper element (106) which lies vertically on an axis (124) in such a manner that it can be rotated relative to a sample (P) which lies horizontally to a fixed position sanitary paper or textile with at least one layer, which acts orthogonally on the sample (P) which is tensioned on a measurement housing (108) with a specifiable penetration force (F), and which deforms the sample (P) and penetrates the sample (P)
- due to a horizontal sample (P) of the sanitary paper or textile with at least one layer, which lies in such a manner that it can be rotated relative to a fixed position scraper element (106) which lies vertically on an axis (124), which acts orthogonally with a specifiable penetration force (F) on the fixed position scraper element (106), as a result of which the scraper element (106) penetrates into the sample (P) which is tensioned on a measurement housing (108) and deforms the sample (P),
within a specified measuring period (Δt), a noise is produced, received and registered, wherein the generated vibrations are evaluated using a vibration analysis and by determining the sound spectrum or a frequency range, and each sound spectrum or frequency range determined is assigned a specific degree of softness of the sample (P).

21. A method according to claim 20, wherein
the vibration analysis is conducted using the sound spectrum or frequency range, using an evaluation of the sound intensity (I) and/or the sound level (Lₚ) and/or the frequencies (f) of the sound pressure (p) in specifiable areas of the sound spectrum and/or the frequency range, and from this, a complex characteristic (K) is calculated which correlates to the softness of the sample (P).

22. A method according to claim 20 and/or 21, wherein
prior to the measurement to determine the softness, a calibration is conducted in such a manner that in each specifiable area of the sound spectrum or the frequency range, one specific softness respectively is assigned to at least one reference sound spectrum or at least one reference frequency range, and to a complex characteristic (K) which can be calculated from this.

23. A method according to claims 20 to 22, wherein the evaluation of the received sound spectra or frequency ranges is conducted using a Fourier analysis by deconstructing the individual vibrations of the noises generated.

24. A method according to claims 20 to 22, wherein
- the impact of the force (F) and the relative movement (Δs) between the sample (P) and the scraper element (106) is conducted simultaneously or in succession and measured
- in the specifiable measuring period (Δt), the noise is received and registered, and
- finally, an evaluation of the noise is conducted using the sound spectrum or frequency range, while determining the characteristic (K).

25. A method according to claims 20 to 24, wherein a determination of the softness is conducted for the purpose of a comparability of the results, preferably under normal conditions, of a temperature (T=23°C) and of a relative humidity (ω = 50%).

26. A method according to claim 20, wherein the specifiable penetration force (F) for sanitary papers lies in the range of approx. 0.01 N to approx. 150 N, and for textiles, in or above this range.

## Revendications

1. Dispositif (100) pour la détermination de la douceur d'un papier hygiénique ou d'un textile avec au moins une couche,
le dispositif comprenant un élément racleur (106) posé verticalement sur un axe (124), lequel élément est disposé de façon pivotante relativement à un échantillon (P) avec au moins une couche du papier hygiénique ou du textile, posé horizontalement en position fixe, et lequel élément étant réalisé de façon déplaçable relativement à l'échantillon (P) tendu sur un boîtier de mesure (108) sous l'application d'une force de pénétration (F) s'exerçant orthogonalement sur l'échantillon (P) le long de l'axe (124) de telle sorte qu'il pénètre dans l'échantillon (P) avec déformation de l'échantillon (P), un capteur de vibrations (116) étant disposé dans la zone des vibrations apparaissant entre l'échantillon (P) et l'élément racleur (106), lequel capteur enregistre les sons produits pendant la rotation de l'élément (106) agissant sur l'échantillon (P) suscitée autour de l'axe (124).

2. Dispositif (100) pour la détermination de la douceur d'un papier hygiénique ou d'un textile avec au moins une couche,
le dispositif comprenant un élément racleur (106) posé verticalement sur un axe (124), lequel élément est disposé en position fixe relativement à un échantillon (P) avec au moins une couche du papier hygiénique ou du textile, posé horizontalement de façon pivotante, et l'échantillon (P) tendu sur un boîtier de mesure (108) étant réalisé de façon déplaçable sous l'application d'une force de pénétration (F) de l'élément racleur (106) s'exerçant orthogonalement sur l'échantillon (P) le long de l'axe (124) de telle sorte que l'élément racleur (106) pénètre dans l'échantillon (P) avec déformation de l'échantillon (P), un capteur de vibrations (116) étant disposé dans la zone des vibrations apparaissant entre l'échantillon (P) et l'élément (106), lequel capteur enregistre les sons produits pendant la rotation de l'élément (106) agissant sur l'échantillon (P) suscitée autour de l'axe (124).

3. Dispositif selon la revendication 1 ou 2,
le capteur de vibrations (116) étant disposé dans le champ acoustique du son, indépendamment ou au niveau du dispositif (100), de préférence au niveau de l'élément racleur (106) lui-même ou dans la zone d'un dispositif de retenue de l'échantillon (P).

4. Dispositif selon la revendication 3,
l'échantillon (P) étant disposé sur le dispositif de retenue avec formation d'un plan d'échantillon (122) essentiellement plan, le dispositif de retenue comprenant essentiellement un boîtier de mesure (108), sur lequel l'échantillon (P) peut être fixé au moyen d'au moins un élément de retenue (114).

5. Dispositif selon les revendications 2 à 4,
le boîtier de mesure (108) présentant au moins une ouverture, sur laquelle l'échantillon (P) est fixé, le boîtier de mesure (108) étant formé en outre de façon facultative partiellement ouvert ou fermé, de telle sorte que, pour un champ acoustique se formant entre l'élément racleur (106) et l'échantillon (P), une zone acoustique ouverte ou un espace acoustique limité (110) peuvent être formés, dans la zone de laquelle ou duquel le capteur de vibrations (116) est disposé.

6. Dispositif selon les revendications 3 à 5,
le capteur de vibrations (116) étant disposé à l'extérieur ou dans la zone du dispositif de retenue ou à l'extérieur ou dans la zone du boîtier de mesure (108).

7. Dispositif selon la revendication 1 ou 2,
un instrument de mesure de force (118) étant disposé pour l'ajustement et le mesurage de la force de pénétration (F) de l'élément racleur (106), lequel instrument est disposé dans la zone du dispositif de retenue de l'échantillon (P) en fonction de la direction de la force de pénétration (F).

8. Dispositif selon la revendication 1 ou 2,
un instrument de mesure de force (118) étant disposé pour l'ajustement et le mesurage de la force de pénétration (F) de l'élément racleur (106), lequel instrument est disposé au niveau de l'élément racleur (106) lui-même ou dans la zone de l'élément racleur (106) en fonction de la direction de la force de pénétration (F).

9. Dispositif selon la revendication 1 ou 2,
un instrument de mesure de trajet (132) étant disposé pour la mesure de la profondeur de pénétration de l'élément racleur (106), par lequel instrument un trajet de déplacement (Δs) de l'élément racleur (106) ou de l'échantillon (P) est mesurable comme paramètre pour l'élasticité de l'échantillon, de façon spécifiable et/ou en fonction du matériau de l'échantillon (P) et de la force de pénétration (F).

10. Dispositif selon la revendication 1 ou 2 ou 7 ou 8,
la force de pénétration (F) de l'élément racleur (106) agissant sur l'échantillon (P) dans chaque angle, de préférence orthogonalement à un plan d'échantillon (122) essentiellement horizontal.

11. Dispositif selon la revendication 1 ou 2,
l'élément mobile respectif, élément racleur (106) ou échantillon (P) est disposé de façon à pouvoir se déplacer en rotation, en translation ou en mouvement pendulaire, avec production du son.

12. Dispositif selon la revendication 1 ou 2,
le mouvement relatif de l'élément racleur (106) ou de l'échantillon (P) d'une part, et le mouvement de déplacement de l'élément racleur (106) ou de l'échantillon (P) pour l'ajustement de la force de pénétration (F) d'autre part ont lieu par un entraînement mécanique ou électrique ou pneumatique ou hydraulique ou similaire, lequel est relié directement ou indirectement par un moyen de transmission (104) avec l'élément (100) ou l'échantillon (P).

13. Dispositif selon la revendication 1 ou 2,
le capteur de vibrations (110) étant un microphone, lequel est relié avec une unité de calibrage et d'évaluation (126), le microphone et/ou l'unité de calibrage et d'évaluation (126) étant disposés dans la zone du dispositif de retenue ou encore à l'intérieur ou à l'extérieur du boîtier de mesure (108).

14. Dispositif selon les revendications 1 à 4,
dans la zone de mesure, dans la zone du dispositif de retenue ou dans le boîtier de mesure (108) ou dans une chambre climatique entourant le dispositif (100), un capteur de température (128) et/ou un capteur d'humidité (130) pour la détermination de la température (T) et/ou de l'humidité relative (ω) sont disposés.

15. Dispositif selon la revendication 1 ou 2,
l'élément racleur (106) présentant une plaque plane comme corps de composant ou étant formé d'une pièce ou de plusieurs pièces avec les éléments fournissant un contour dirigés vers l'échantillon (P), de telle sorte que différentes formes (106D) peuvent être formées vis-à-vis du plan d'échantillon (122), lesquelles forment des surfaces de contact planes ou concaves ou convexes ou en pointe.

16. Dispositif selon la revendication 15,
une rugosité de surface définie étant spécifiable sur la surface de l'élément (106) (surface de contact) dirigée vers l'échantillon (P).

17. Dispositif selon la revendication 1,
des motifs de papier hygiénique ou des motifs textiles identiques ou différents relativement à l'échantillon (P) étant disposés sur la surface de l'élément racleur (106) (surface de contact) dirigée vers l'échantillon (P), une rugosité de surface définie étant également spécifiable.

18. Dispositif selon la revendication 1 ou 2,
l'élément racleur (106) se composant de différents matériaux de dureté minimale appropriée, des plastiques ou des métaux avec rugosités de surface spécifiables étant employés de préférence.

19. Dispositif selon la revendication 4,
l'échantillon (P) étant disposé sur le dispositif de retenue avec formation du plan d'échantillon (122) essentiellement plan, l'échantillon (P) faisant appui sur une feuille ou une plaque fermée ou partiellement munie d'ouvertures, avec des épaisseurs de matériau et/ou des propriétés de matériau différemment réalisables.

20. Dispositif pour la détermination de la douceur d'un papier hygiénique ou d'un textile avec au moins une couche,
- par un élément racleur (106) posé verticalement sur un axe (124) de façon pivotante relativement à un échantillon (P) avec au moins une couche du papier hygiénique ou du textile, posé horizontalement en position fixe, lequel élément agit orthogonalement sur l'échantillon (P) tendu sur un boîtier de mesure (108) avec une force de pénétration (F) spécifiable et déforme l'échantillon (P) et pénètre dans l'échantillon (P),
ou
- par un échantillon (P) avec au moins une couche du papier hygiénique ou du textile, posé horizontalement de façon pivotante relativement à un élément racleur (106) en position fixe posé verticalement sur un axe (124), lequel échantillon agit orthogonalement avec une force de pénétration (F) spécifiable sur l'élément racleur (106) en position fixe, par quoi l'élément racleur (106) pénètre dans l'échantillon (P) tendu sur un boîtier de mesure (108) et déforme l'échantillon (P),
un son étant produit, reçu et enregistré dans un intervalle de mesure spécifiable (Δt), les vibrations produites étant évaluées au moyen d'une analyse de vibrations, par la détermination d'un spectre sonore ou d'une bande de fréquences, et une douceur donnée de l'échantillon (P) étant attribuée à chaque spectre sonore ou à chaque bande de fréquences déterminés.

21. Procédé selon la revendication 20,
l'analyse de vibrations étant effectuée par le spectre sonore ou la bande de fréquences, par l'évaluation de l'intensité sonore (I) et/ou du niveau sonore (Lp) et/ou des fréquences (f) de la pression acoustique (p) dans les plages spécifiables du spectre sonore et/ou de la bande de fréquences, et un indicateur complexe (K) étant calculé à partir de ceux-ci, lequel est en corrélation avec la douceur de l'échantillon (P).

22. Procédé selon la revendication 20 et/ou 21,
un calibrage étant effectué avant la mesure pour la détermination de la douceur de telle sorte que, dans une plage spécifiable du spectre sonore ou de la bande de fréquences, une douceur donnée est attribuée respectivement à un spectre sonore de référence ou au minimum à une bande de fréquences de référence et à un indicateur complexe (K) calculable à partir de ceux-ci.

23. Procédé selon les revendications 20 à 22,
l'évaluation des spectres sonores ou des bandes de fréquences reçus étant exécutée par la décomposition des différentes vibrations des sons produits au moyen de l'analyse de Fourier.

24. Procédé selon les revendications 20 à 22,
- l'action de la force (F) et le mouvement relatif (Δs) entre l'échantillon (P) et l'élément racleur (106) étant effectués et mesurés simultanément ou l'un après l'autre,
- le son étant reçu et enregistré dans l'intervalle de mesure spécifiable (Δt), et
- finalement, une évaluation du son étant calculée au moyen du spectre sonore ou de la bande de fréquences, par la détermination de l'indicateur (K).

25. Procédé selon les revendications 20 à 24,
une détermination de la douceur pour la comparabilité des résultats étant exécutée de préférence sous des conditions normales de température (T=23°C) et d'humidité relative (w = 50 %).

26. Procédé selon la revendication 20,
la force de pénétration (F) spécifiable se trouvant dans la plage d'environ 0,01 N à environ 150 N pour les papiers hygiéniques, et dans cette plage et au-delà pour les textiles.
